# EUROPEAN PATENT SPECIFICATION

(11) **EP 1 051 401 B1**
(45) Date of publication and mention of the grant of the patent: **24.10.2007**
(21) Application number: 98956918.1
(22) Date of filing: 10.11.1998
(51) Int. Cl.: C07D 233/68

(54) **PROCESS FOR THE PRODUCTION OF FORMYLIMIDAZOLES**
VERFAHREN ZUR HERSTELLUNG VON FORMYLIMIDAZOLEN
PROCEDE DE PRODUCTION DE FORMYLIMIDAZOLES

(30) Priority: 14.11.1997 CH 263897; 27.11.1997 CH 273897
(43) Date of publication of application: 15.11.2000
(73) Proprietor: Lonza AG, CH-3945 Gampel/Wallis (CH)
(72) Inventor: BESSARD, Yves, CH-Basel (CH); HEVELING, Josef, CH-Basel (CH)
(74) Representative: Waters, David Martin
(86) International application number: PCT/EP1998/007322
(87) International publication number: WO 1999/025696

(56) References cited:
- CH-A- 685 496
- S. UESATO ET AL.: "Studies on Monoterpene Glucosides and Related Natural Products. XVVII. Synthesis of 3H- and 2H-labeled Iridodial Derivatives for Studies on the Biosynthesis of Iridoid Glucosides" CHEM. PHARM. BULL., vol. 30, no. 11, 1982, pages 3942-3950, XP002092318
- J. D. WHITE ET AL.: "Synthesis of Curacin A: A Powerful Antimitotic from the Cyanobacterium Lyngbya majuscula" J. AM. CHEM. SOC., vol. 117, no. 20, 1995, pages 5612-5613, XP002092319
- R. SHIOZAKI ET AL.: "H2O2 Oxidation by Ce(IV) contained weakly-type heteropolyoxometalate for various alcohols" SYNTH. COMM., vol. 26, no. 9, 1996, pages 1663-1668, XP002092320
- T. MORIMOTO ET AL.: "Oxidation of Alcohols to Carbonyl Compounds with Peracetic Acid catalysed by Cobalt(III) Acetate" J. CHEM. SOC. PERKIN TRANS. 2,1984, pages 1949-1952, XP002092321
- S. P. SRIVASTAVA, V. K. GUPTA: "Kinetics and Mechanism of Ag+ Catalysed Oxidation of Alcohols by Peroxodisulphate Ion in Aqueous Medium" J. INDIAN CHEM. SOC., vol. 57, no. 8, 1980, pages 797-799, XP002092322
- H. SUGIMOTO, D. T. SAWYER: "Iron(II)-Induced Activation of Hydroperoxides for the Dehydrogenation and Monooxygenation of Organic Substrates in Acetonitrile" J. AM. CHEM. SOC., vol. 107, no. 20, 1985, pages 5712-5716, XP002092323
- P. MÜLLER, H. IDMOUMAZ: "Rhodium-catalyzed oxidation of organic compounds with t-butyl hydroperoxide" J. ORGANOMET. CHEM., vol. 345, 1988, pages 187-199, XP002092324

## Description

This invention concerns a new procedure for producing formyl imidazoles of the general formula 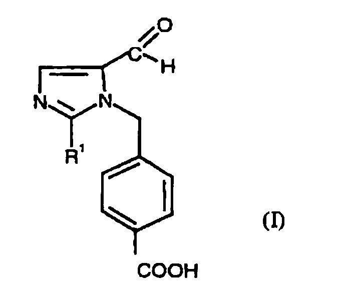 in which R¹ is C₁₋₆alkyl, by catalytic oxidation of hydroxy methyl imidazoles of the general formula 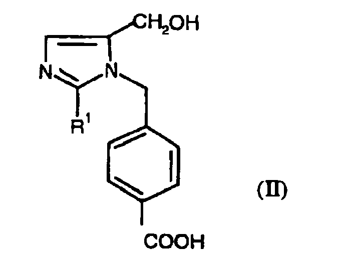 in which R¹ has the meaning given above.

Formyl imidazoles are important intermediate products, for example, for the production of pharmaceutical substances such as diuretics or antihypertensives (WO-A 92/20651). Several procedures have previously been known for producing formyl imidazoles. In CH-A 685496, a procedure is described in which the catalytic oxidation of hydroxy methyl imidazoles to formyl imidazoles is performed in the presence of noble metal catalysts such as platinum bismuth, platinum black, platinum or palladium on activated charcoal with oxygen insufflation.

The task of the invention was therefore to make available an economical, improved procedure for producing formyl imidazoles.

In this invention, this task is solved by the procedure defined in Claim 1. Accordingly, the present invention provides a process for producing formyl imidazoles of the formula (I): 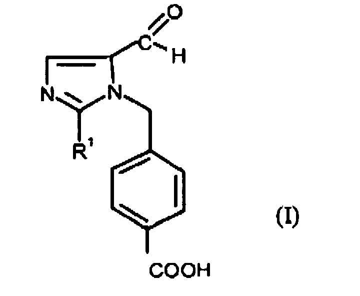 in which R¹ is C₁₋₆ alkyl, which comprises catalytic oxidation of hydroxymethyl imidazoles of the general formula (II): 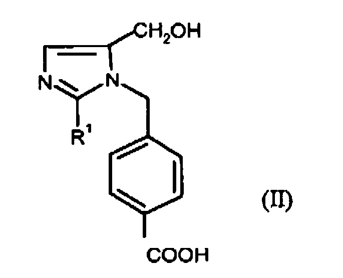 in which R¹ is as defined above, in the presence of a noble metal catalyst selected from a platinum/bismuth catalyst or a platinum/lead catalyst,
wherein that the catalytic oxidation takes place in the presence of a peroxide selected from hydrogen peroxide and in presence of water, a water-miscible solvent or mixtures thereof, in an alkaline medium,
wherein the alkaline medium is obtained by adding an alkali hydroxide, an alkali carbonate or an alkali acetate to the reaction mixture; and
wherein the reaction is performed at a temperature of 20° to 120°C.

R¹ is a straight-chained or ramified alkyl group with 1 to 6 C atoms. Specifically, this may be methyl, ethyl, propyl, isopropyl, butyl, isobutyl, tert-butyl, pentyl and its isomers as well as hexyl and its isomers. The preferred meaning R¹ is butyl.

Hydroxy methyl imidazoles can be readily produced as starting compounds as specified, for example, in WO-A 92/20651 or in E.F. Godefroi et al., Trav. Chim. Receuil Pays-Bas, 91, 1383 (1972).

The noble metal catalyst is used by itself or bound to a vehicle such as, for example, activated charcoal, silicon dioxide, aluminum dioxide, silicon-aluminum dioxide, zirconium oxide or titanium oxide. It is preferably bound to activated charcoal.

Noble metal catalysts bound to activated charcoal can be commercially obtained, for example, from Degussa.

The appropriate percentage of the noble metal bound to a vehicle is between 0.1 and 15% by weight, and preferably between 0.5 and 7% by weight, relative to the vehicle material.

The noble metal catalyst is preferably used in an amount of 0.05 to 1.0 mol % noble metal base relative to hydroxy methyl imidazole, and an amount of 0.1 to 0.4 mol % noble metal base relative to hydroxy methyl imidazole is especially preferred. Hydrogen peroxide used in a 10% to 30% aqueous solution is particularly suitable.

The catalytic oxidation takes place appropriately in the presence of water, a water-miscible solvent or mixtures thereof, in an alkaline milieu.

Particularly suitable water-miscible solvents are, for example, alcohols or carboxylic acids with 1 to 6 C atoms or ketones such as, for example, acetone or methyl ethyl ketone.

Mixtures of water and water-miscible solvents are preferably used. It has proven to be advantageous if the water used is adjusted to be alkaline, by the appropriate addition of an alkali hydroxide, an alkali carbonate or an alkali acetate. Alkali hydroxide is preferably used in the ratio 1: 0.05 to 5, and preferably 1: 1 to 3, relative to the mol amount used of the hydroxy methyl imidazole of general formula II.

The catalytic oxidation is appropriately performed at a temperature of 20° to 120°C, preferably at 50° - 80°C.

After the standard peroxide dosing time of 2 -3 hours, the compound of general formula I can be isolated in the standard manner after a sufficient secondary reaction time.

The product is isolated by appropriate crystallization and filtration. The catalyst used can be used several times with no loss of activity.

### Examples

### Example 1

### Production of 4-[(2-butyl-5-formyl- 1H-imidazo-1-yl)methyl]benzoic acid

9.5 g (33 mmol) 4-[(2-butyl-5-hydroxymethyl-1H-imidazo-1-yl)methyl]benzoic acid, 40 ml water, 10 ml methanol, 4.2 g (105 mmol) NaOH and 0.92 g 5% platinum and 5% bismuth on activated charcoal (containing 60% water) are placed in a 100-ml flask at room temperature and heated to 60°C. 6.6 g (39 mmol) 20% aqueous H₂O₂ solution were added to this suspension at 60°C over 60 minutes and the mixture was then converted with HPLC. 6.6 g (39 mmol) 20% aqueous H₂O₂ solution were again added over 60 minutes. 1.7 g (10 mmol) 20% aqueous H₂O₂ solution were then added over 20 minutes (conversion > 90%). The mixture was cooled to room temperature. After acidification to pH 6.0 with 17.6 ml HCl (15%), the product was preciptated. It was cooled to 2°C, filtered, washed with 2 x 20 ml water, and dried at room temperature at 15 mbar. 7.2 g (70%) of yellow 4-[(2-butyl-5-formyl-1H-imidazo-1-yl)methyl]benzoic acid (HPLC content 95%) were obtained.
Melting point: 144 - 146°C.

| | |
|---|---|
| ¹H-NMR (DMSO-_{d6}, 400 MHz)δ: | 12.9 (1H, s); |
| | 9.65 (1H, s); |
| | 7.94 (1H, s); |
| | 7.90 (2H, d); |
| | 7.11 (2H, d); |
| | 5.65 (2H, s); |
| | 2.63 (2H, t); |
| | 1.54 (2H, pent); |
| | 1.36 (2H, hex); |
| | 0.79 (3 H, t). |

## Claims

1. A process for producing formyl imidazoles of the formula (I): 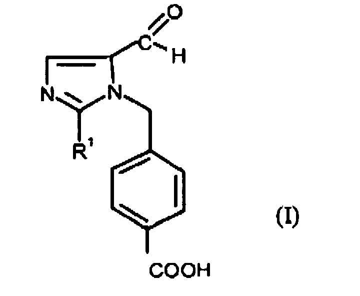 in which R¹ is C₁₋₆ alkyl, which comprises catalytic oxidation of hydroxymethyl imidazoles of the general formula (II): 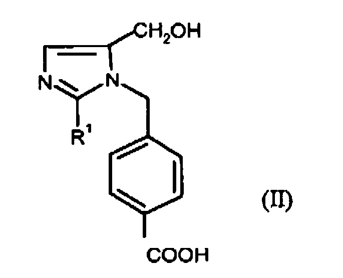 in which R¹ is as defined above, in the presence of a noble metal catalyst selected from a platinum/bismuth catalyst or a platinum/lead catalyst,
wherein that the catalytic oxidation takes place in the presence of a peroxide selected from hydrogen peroxide and in presence of water, a water-miscible solvent or mixtures thereof, in an alkaline medium,
wherein the alkaline medium is obtained by adding an alkali hydroxide, an alkali carbonate or an alkali acetate to the reaction mixture; and
wherein the reaction is performed at a temperature of 20° to 120°C.

2. Process as in Claim 1, wherein R¹ is butyl.

## Patentansprüche

1. Verfahren zur Herstellung von Formylimidazolen der Formel (I): 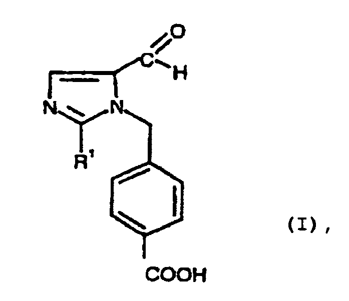 worin R¹ C₁₋₆-Alkyl ist, welches die katalytische Oxidation von Hydroxymethylimidazolen der allgemeinen Formel (II) : 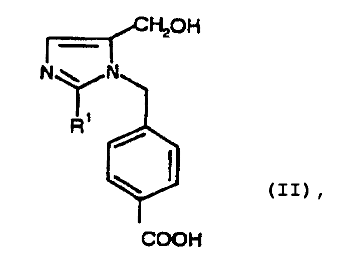 worin R¹ wie oben definiert ist, in der Gegenwart eines Edelmetall-Katalysators, ausgewählt aus einem Platin/Wismut- oder einem Platin/Blei-Katalysator, umfasst,
worin die katalytische Oxidation in der Gegenwart eines Peroxids, ausgewählt aus Wasserstoffperoxid, und in der Gegenwart von Wasser, einem mit Wasser mischbaren Lösungsmittel oder von Mischungen davon, in einem alkalischen Medium erfolgt,
worin das alkalische Medium durch Zugabe von Alkalihydroxid, Alkalicarbonat oder Alkylacetat zur Reaktionsmischung erhalten wird, und
worin die Reaktion bei einer Temperatur von 20 bis 120°C durchgeführt wird.

2. Verfahren gemäß Anspruch 1, worin R¹ Butyl ist.

## Revendications

1. Procédé de production de formylimidazoles de formule (I) : 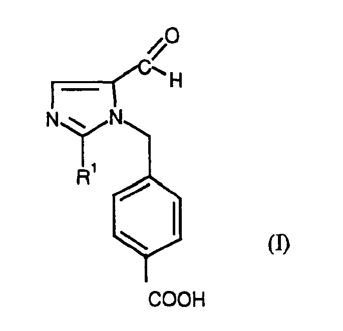 dans laquelle R¹ est un groupe alkyle en C₁-C₆, qui comprend une oxydation catalytique d'hydroxyméthylimidazoles de formule générale (II) : 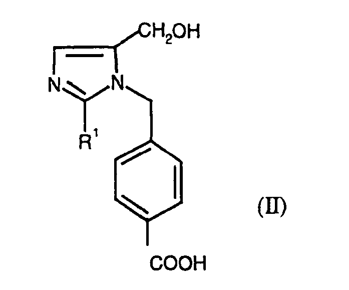 dans laquelle R¹ est comme défini ci-dessus, en présence d'un catalyseur de métal noble choisi parmi un catalyseur de platine/bismuth ou un catalyseur de platine/plomb,
dans lequel l'oxydation catalytique a lieu en présence d'un peroxyde choisi parmi le peroxyde d'hydrogène et en présence d'eau, d'un solvant miscible dans l'eau ou de mélanges de ceux-ci, dans un milieu alcalin,
dans lequel le milieu alcalin est obtenu en ajoutant un hydroxyde alcalin, un carbonate alcalin ou un acétate alcalin au mélange réactionnel ; et
dans lequel la réaction est réalisée à une température de 20 °C à 120 °C.

2. Procédé selon la revendication 1, dans lequel R¹ est un groupe butyle.
